# EUROPEAN PATENT APPLICATION

(11) **EP 4 242 201 A1**
(43) Date of publication of application: **13.09.2023**
(21) Application number: 22161722.8
(22) Date of filing: 11.03.2022
(51) Int. Cl.: C07D 311/78, B01D 15/26, B01D 15/40, C07C 37/00, C07C 37/82, C07C 39/23

(54) **CANNABINOIDS EXTRACTION AND CONVERSION**

(71) Applicant: Bridge Farm Nurseries Limited, Spalding, Lincolnshire PE11 2TA (GB)
(72) Inventor: Marriott, Ray, Kettering, NN16 8TZ (GB)
(74) Representative: Tollett, Ian

(57) **Abstract**

The present invention discloses a method for extracting cannabidiol from milled and decarboxylated hemp using subcritical liquid carbon dioxide and for converting the extracted cannabidiol to tetrahydrocannabinol using supercritical carbon dioxide in a sequential process.

## Description

### Field of the invention.

The present invention discloses a method for extracting cannabidiol from hemp using subcritical liquid carbon dioxide and converting the cannabidiol to tetrahydrocannabinol in a sequential process.

### Introduction

For pharmaceutical use it is desirable to have a mixture of cannabidiol (CBD) and tetrahydrocannabinol (THC) to treat certain conditions and this can be achieved by selecting specific *Cannabis sativa* or *Cannabis indica* strains with known ratios of these cannabinoids and if necessary, extracting the cannabinoids from decarboxylated biomass using a suitable solvent to obtain an extract with known ratios of CBD and THC. However, this results in an extract with a complex mixture of cannabinoids and other molecules in addition to CBD and Δ⁹-THC.

It is known that cannabidiol isomerises to delta-9-tetrahydrocannabinol (Δ⁹-THC) and other cannabinoids under aqueous acidic conditions resulting in a mixture of isomeric tetrahydrocannabinols in low yield. More recently, in patented methods, CBD was converted to Δ⁹-THC in quantitative yield.

For example, WO02/070506 to Webster et al. discloses a method for converting CBD to THC using respectively p-toluenesulfonic acid as Lewis acid in anhydrous dichloromethane for the conversion of CBD into Δ⁸-THC and boron trifluoride etherate for the conversion into Δ⁹-THC. The conversion was carried out under nitrogen atmosphere. The starting material was CBD. That document does not disclose the extraction of CBD from hemp.

US2020/0255389 to Tegen et al. discloses the purification of Δ⁹-THC using various solvents. It further discloses the conversion of CBD to THC sing a solvent such as dichloromethane and a Lewis acid such as p-toluenesulfonic acid.

US9,959,976 to Keller discloses a process for extracting CBD from hemp and converting it into THC. The extraction was carried out using dichloromethane in combination with ethanol. The conversion was subsequently carried out by mixing the extracted cannabidiol in ethanol and dichloromethane with sulphuric acid. The cannabinoid fractions were then separated in a fractionation unit.

It is also known that silica can catalyse certain reactions as was disclosed for example in Weinstein et al. (Journal of Physical Chemistry B, 1999, 103 (15), 2878-2887). That documents demonstrated that silica in supercritical CO₂ could catalyse Diels-Alder reactions including the formation of 6-carbon rings by cycloaddition.

Romanenko and Tkachev (Chemistry for Sustainable Development, 2007, 15(5), 571-585) showed that both acidified silica and alumina could catalyse the rearrangement of caryophyllene oxide including double bond migration. Silica was a much more active than alumina.

There is a need to provide hemp extracts that contain mixtures of CBD and THC in a known ratio suitable for use in pharmaceutical preparations and it would be preferable to be able to use a CBD rich hemp variety that can be grown without regulatory restrictions and be able to control the CBD and THC ratio in the final extract using an extraction process that simultaneously extracts and converts the CBD to Δ⁹-THC without the use of chemical conversion agents.

### Summary of the invention.

It is an object of the present invention to extract cannabidiol (CBD) from hemp with carbon dioxide.

It is also an object of the present invention to sequentially extract CBD from biomass and convert the CBD to THC.

It is a further object of the present invention to provide a simple and cost-effective method for extracting and converting CBD to THC in a fixed ratio.

The foregoing objectives have been realised as described in the independent claims. Preferred embodiments are described in the dependent claims.

### List of Figures.

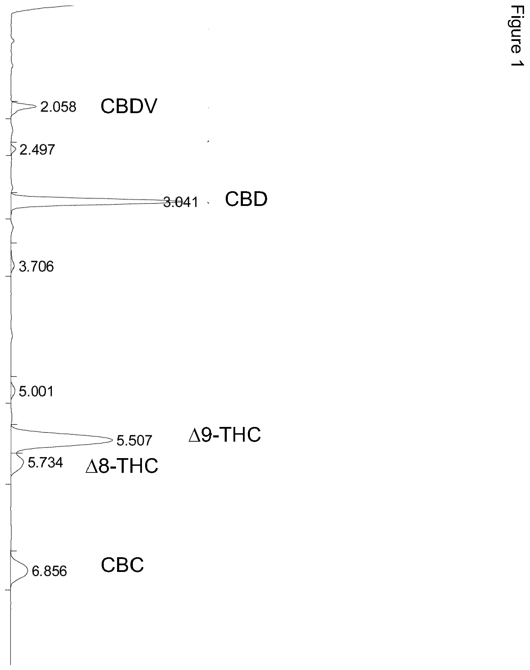

Figure 1 represents the High Precision Liquid Chromatography (HPLC) trace showing the cannabinoids present on the recovered silica.

### Detailed description of the invention.

Accordingly, the present invention discloses a method for extracting cannabidiol from hemp and converting the cannabidiol to tetrahydrocannabinol that comprises the steps of:
a) Providing 2 vessels, vessel 1 and vessel 2, wherein vessel 2 is connected to vessel 1, and a separator vessel connected to vessel 2;
b) Milling dried hemp biomass in order to obtain a product having a bulk density of at least 0.2 g/cm³;
c) Decarboxylating the dried biomass of step b)
d) Filling vessel 1 with the milled and decarboxylated biomass of step c)
e) Filling vessel 2 with silica gel, selected from narrow pore gels or from wide pore gels, and for its ability to bind resorcinols and phenols :
f) In vessel 1, extracting a product comprising predominantly cannabidiol with other minor cannabinoids from the milled and decarboxylated biomass by injecting subcritical liquid carbon dioxide at a pressure of at least 65 bars and a temperature of less than 31°C, with a mass ratio carbon dioxide to biomass of at least 10 kg CO₂ /kg biomass and for a period of time ranging between 30 minutes and 8 hours;
g) Continuously flowing the extraction product of step f) into vessel 2 filled with silica gel wherein said vessel 2 is submitted to the same flow of subcritical liquid carbon dioxide as vessel 1, that is, the same mass ratio carbon dioxide to biomass, the same temperature, the same pressure and the same period of time as in vessel 1;
h) Retrieving and separating terpenes and waxes in the separator vessel connected to vessel 2;
i) In vessel 2, converting cannadidiol to tetrahydrocannabinol by submitting said vessel 2 to a flow of supercritical carbon dioxide at a mass ratio carbon dioxide to biomass of at least 10 kg CO₂/kg biomass and for a period of time ranging between 30 minutes and 8 hours, and:
j) Sequentially recovering all products in the separator vessel said products comprising in order of recovery CBD, Δ9-THC and Δ8-THC, and other minor cannabinoids.

The milling of dried biomass can be carried out either with a hammer mill or with a knife mill. The hammer mill produces a coarse milled material having a bulk density of a little over 0.2 g/cm³, whereas the knife mill can produce finer milled material of at least 0.3 g/cm³. Optionally the milled biomass can be pelleted to increase the bulk density to over 0.6g/cm³. The choice of milling and pelleting method has an economic impact: coarse milled material tends to present a greater barrier to the diffusion of CO₂ into the biomass thereby extending the extraction time.

The decarboxylation step is needed in order to convert CDBA into CBD before processing takes place. The decarboxylation is carried out by heating the milled biomass to eliminate one mole of carbon dioxide.

Decarboxylation is a well-known process which is temperature and time dependent. Several trials carried out in the present work have established that the temperature necessary for a high conversion of CDBA into CBD is of at least 100°C for a period of time of at least 1 hour. The operating conditions cannot however be too severe and must be selected in order to minimise degradation of the final products. Preferably, the decarboxylation step is carried out at a temperature ranging between 100 and 200°C, more preferably between 110 and 130°C. Preferably it is carried out for a period of time ranging between 1 and 3 hours, more preferably between 1.5 and 2.5 hours. The decarboxylation can be carried out either in a moving bed (drum dryer/heater) or in a vacuum oven. The overall mass loss observed during the process, of less than 20 mass%, is mostly due to reduction in moisture, loss of carbon dioxide through the decarboxylation process and loss of a small quantity of monoterpenes. The decarboxylated milled biomass, rich in CBD, is then submitted to the extraction step.

The decarboxylation step is an important feature of the present process. If it is not carried out before the extraction step, both the CBD and THC exist as their acid forms CBDA and THCA respectively. This means that both molecules carry one active carboxyl group which makes the present extraction and conversion technique impossible.

The acid forms CBDA and THCA are much less soluble in subcritical liquid carbon dioxide than the neutral CBD and THC forms, thereby requiring increased pressure and temperature to carry out the extraction. In the present method, the extraction is carried out at low pressure and temperature with subcritical liquid carbon dioxide. This leaves the CBDA behind, extracting only CBD.

The support used in the second vessel is selected according to its ability to bind both resorcinol-type products such as CBD and phenol-type products such as THC and carry out the conversion of CBD to THC. The mechanism is based on the dual ability of the support to first bind the resorcinols and phenols by the physical process of hydrogen bonding to the silica surface. The conversion is then carried out by a chemical process at the surface of the silica gel wherein the bound water is acidified by the CO₂, thereby decreasing its pH to as low as 3, which catalyses the conversion. The water comes from the extracted biomass: there is typically 5-8% water when extracted and even though water is not very soluble in CO₂ there is a sufficient amount of water co-extracted to decrease the pH. The conversion reaction is shown below, wherein Δ-9-THC is the main product. It can isomerise to Δ-8-THC which we see to a small extent.

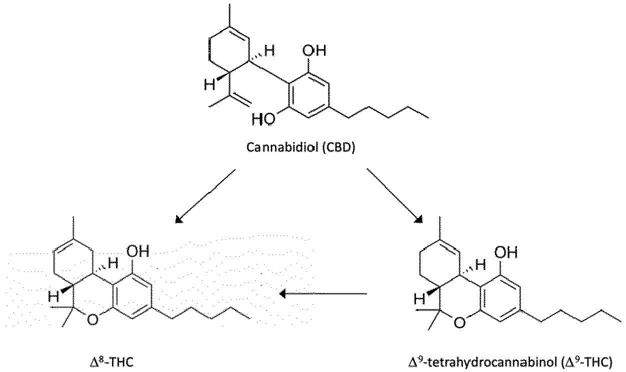

Under liquid CO₂ conditions the non-polar terpenes and waxes are not bound to the silica gel: they pass through vessel 2 and are collected in the separator.

The support in vessel 2 is a silica gel more properly known as a silica xerogel which has an average pore size of 2.4 nanometres in the case of narrow pore gels and 11 nanometres in the case of large pore gels. The preferred silica gel of the present invention is the narrow pore gel having an average pore size of 2.4 nm, a high surface area of around 750-800m²/g, a bulk density ranging between 0.5 g/cm³ and 0.85 g/cm³, depending on whether it is granular or beaded, granular being at the lower end of the range. Granular product is preferably selected in the present invention with a density of approximately 0.6 g/cm³. The particle size ranges between 200 and 500µm.

The extraction of cannabidiol from the milled and decarboxylated biomass is carried out in vessel 1 by injecting subcritical liquid carbon dioxide at a pressure of at least 65 bars and a temperature of less than 31°C, with a mass ratio carbon dioxide to biomass of at least 10 kg CO₂ /kg biomass and for a period of time ranging between 30 minutes and 8 hours;

Preferably, the pressure is of at least 90 bars and most preferably of at least 100 bars, and the temperature is less than 25 °C and most preferably less than 20 °C. Preferably the mass ratio of carbon dioxide to biomass is of at least 20 Kg CO₂/kg biomass and more preferably at least 25 Kg CO₂/kg biomass.

The extraction product of step f) is continuously flowed into vessel 2 filled with silica gel, simultaneously with to the flow of subcritical liquid carbon dioxide exiting vessel 1, that is, the same mass ratio carbon dioxide to biomass, the same temperature, the same pressure and the same period of time as in vessel 1.

The terpenes and waxes, which do not bind to the silica gel travel straight through vessel 2 and are collected in the separator vessel connected to vessel 2.

The conversion of CBD to THC is then carried out by submitting vessel 2 to a flow of supercritical carbon dioxide at a mass ratio carbon dioxide to silica of at least 10 kg CO₂/kg silica and for a period of time ranging between 30 minutes and 8 hours. The flow of supercritical carbon dioxide is connected directly to vessel 2.

The conversion process is carried out with supercritical carbon dioxide at a pressure of at least 150bar, preferably between 250bar and 350bar and most preferably of about 300bar and at a temperature of between 40°C and 70°C , preferably of about 50°C.

The mass ratio of carbon dioxide to silica of at least 10 kg CO₂/kg biomass, preferably of between 15 and 20kg CO₂/kg silica and the process is run for a period of time ranging between 30 minutes and 8 hours, preferably about 4 hours

All products are sequentially collected in the separator vessel, said products comprising in order of recovery CBD, Δ9-THC and Δ8-THC. Some other minor cannabinoids are also retrieved in the separator vessel.

It is the objective of the present process to extract a minimum of 95% of the CBD from the biomass and to obtain an extract in which the CBD to THC ratio is reduced from a starting ratio of about 20:1 to a final ratio of about 3:1.

### Examples.

The hemp used in this example was CC clone grown and dried on our research farm. The dried biomass was milled using an Ecohammer 158SP mill and decarboxylated at a temperature of 115 °C for 2 hours in an OV-12 oven with a vacuum extraction after one hour for 5 minutes. Liquid CO₂ was obtained from Air products and the chromatographic support from GeeJay Silicas (UK). All analytical reagents were obtained from Fisher. The decarboxylated biomass had a CBD content of 5.8% and a THC content of 0.2%. In each case both vessels were packed to within 25mm of the top and capped with cotton wool to prevent the filters blinding.

### Example 1.

In the first trial, using the GeeJay silica gel, 479 g of biomass was placed in a 2000 ml vessel 1 and extracted with 15kg CO₂/kg biomass over a period of time of 3-hour, at a pressure of 110 bar and a temperature of 15 °C with the exit of vessel 1 connected to the top of vessel 2 containing the silica gel. After 3 hours the flow was stopped and the separator opened to examine the extract collected over this period. Only 0.16g of a white powder was collected which contained predominantly plant waxes and terpenes with only a small amount of (0.98%) of CBD. Samples of the extracted biomass were collected from the top and bottom of the vessel 1 for analysis. A CO₂ flow at 40g/min was then connected to vessel 2 containing the silica gel and run at a pressure of 300 bar and a temperature of 50°C to collect the extract absorbed on the silica. The fraction weights and analysis are shown in Table 1.

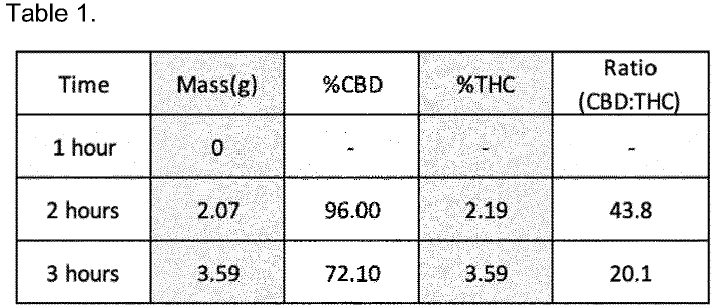

It can be seen that, under such conditions, there was no separation of the CBD and THC and that the level of THC increased as the elution progressed. The elution was stopped after 3 hours and the vessel was depressurised. The silica gel was collected from the top, middle and bottom of the vessel and analysed to yield the results shown in Table 2.

**Table 2.**

| | %CBD | %THC | Ratio (CBD:THC) |
|---|---|---|---|
| Top | 0.32 | 0 | - |
| Middle | 0.83 | 3.79 | 0.219 |
| Bottom | 0.66 | 4.01 | 0.165 |

It can be seen that there is active conversion of CBD to THC on the surface of the silica. It is believed that said conversion results from water bound to the surface of the silica gel that dissolves CO₂ thereby creating acidic centres.

### Example 2.

In this second example, the GJ silica gel of example 1 was dried for 24 hours at a temperature of 125 °C. Using that dried support, 474 g of biomass was extracted in a 2000 ml vessel 1 with 25kg CO₂/kg biomass over a period of time of 5 hours at a pressure of 110 bar and a temperature of 15 °C, with the exit of vessel 1 connected to the top of vessel 2 containing the silica. After 5 hours the flow was stopped and the separator opened to examine the extract collected over this period. Only 0.36g of a white powder was collected which was predominantly plant waxes and terpenes. Samples of the extracted biomass were collected from the top and bottom of the 2000 ml vessel 1 for analysis. The CO₂ flow of 40g/min was then connected to vessel 2 containing the silica and run at a pressure of 300 bar and a temperature of 50 °C to collect the extract absorbed on the silica. The fraction weights and analysis are shown in Table 3.

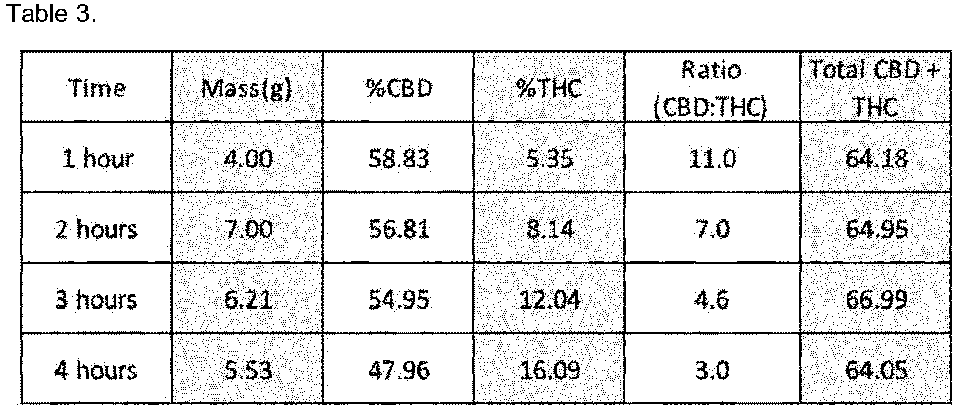

It is clear that the silica gel used, even after drying, catalysed the isomerisation of CBD to THC. This is evidenced by the observation that the cumulative level of the 2 compounds remained unchanged while the CBD/THC ratio dropped in each sequential fraction.

After 4 fractions had been collected, the vessel was depressurised and silica samples collected from the top, middle and bottom of the vessel were analysed to yield the results shown in Table 4.

**Table 4.**

| | %CBD | %THC | Ratio (CBD:THC) |
|---|---|---|---|
| Top | 0.12 | 0.15 | 0.800 |
| Middle | 1.38 | 1.49 | 0.926 |
| Bottom | 2.13 | 2.7 | 0.789 |

The silica was pigmented throughout with the highest concentration of pigment at the bottom of the vessel indicating that the support was strongly binding the carotenoid pigments found in the hemp. In this trial high levels of THC were again observed on the silica after collecting the fractions. In total 6.75 g of THC were recovered: this was 7 times the amount of 0.95 g present in the starting biomass. In addition, Δ9-THC and Δ8-THC were also present together with low levels of other cannabinoids suggesting that the CBD is undergoing a series of isomerisation reactions on the silica surface. Figure 1 shows the HPLC trace from the recovered silica showing the array of cannabinoids found.

## Claims

1. A method for extracting cannabidiol from hemp and converting said cannabidiol to tetrahydrocannabinol that comprises the steps of:
a) Providing 2 vessels, vessel 1 and vessel 2, wherein vessel 2 is connected to vessel 1, and a separator vessel connected to vessel 2;
b) Milling dried hemp biomass in order to obtain a product having a bulk density of at least 0.2 g/cm³;
c) Decarboxylating the dried biomass of step b);
d) Filling vessel 1 with the milled and decarboxylated biomass of step c);
e) Filling vessel 2 with silica gel, selected from narrow pore gels or from wide pore gels, and for its ability to bind resorcinols and phenols;
f) In vessel 1, extracting a product comprising predominantly cannabidiol with other minor cannabinoids from the milled and decarboxylated biomass by injecting subcritical liquid carbon dioxide at a pressure of at least 65 bars and a temperature of less than 31°C, with a mass ratio carbon dioxide to biomass of at least 10 kg CO₂ /kg biomass and for a period of time ranging between 30 minutes and 8 hours;
g) Continuously flowing the extraction product of step f) into vessel 2 filled with silica gel wherein said vessel 2 is submitted to the same flow of subcritical liquid carbon dioxide as vessel 1, that is, the same mass ratio carbon dioxide to biomass, the same temperature, the same pressure and the same period of time as in vessel 1;
h) Retrieving and separating terpenes and waxes in the separator vessel connected to vessel 2;
i) In vessel 2, converting cannabidiol to tetrahydrocannabinol by submitting said vessel 2 to a flow of supercritical carbon dioxide at a mass ratio carbon dioxide to biomass of at least 10 kg CO₂/kg biomass and for a period of time ranging between 30 minutes and 8 hours, and:
j) Sequentially recovering all products in the separator vessel said products comprising in order of recovery CBD, Δ9-THC and Δ8-THC, and other minor cannabinoids.

2. The method of claim 1 wherein the decarboxylation is carried out at a temperature ranging between 100 and 200 °C and during a period of time ranging between 1 and 3 hours.

3. The method of claim 1 or claim 2 wherein the silica gel is selected from narrow pore gel having a average pore size of 2.4 nanometres, a high surface area of at least 750 m²/g, a bulk density of about 0.6g/cm³ and a particle size ranging between 200 and 500µm.

4. The method of any one of the preceding claims wherein the pressure for extraction step f) of claim 1 is of at least 90 bars, preferably of at least 100 bars.

5. The method of any one of the preceding claims wherein the temperature in extraction step f) of claim 1 is of at most 25 °C, preferably of at most 20 °C.

6. The method of any one of the preceding claims wherein the carbon dioxide to biomass ration in extraction step f) is of at least 25 kg CO₂/kg biomass preferably of at least 30 kg CO₂/kg biomass.

7. The method of any one of the preceding claims wherein in step i) the conversion of CBD to THC is carried out with supercritical carbon dioxide at a pressure of at least 150bar and a temperature of at least 40°C and for a period of time of at least 1 hour

8. The method of claim 7 wherein the supercritical carbon dioxide is at a pressure ranging between 150bar and 350bar and a temperature ranging between 40°C and 70°C and a period of time between 1 hour and 4 hours.
